# FASCICULE DE BREVET EUROPEEN

(11) **EP 3 526 594 B1**
(45) Date de publication et mention de la délivrance du brevet: **24.04.2024**
(21) Numéro de dépôt: 17792003.0
(22) Date de dépôt: 16.10.2017
(51) Int. Cl.: G01N 27/414, G01N 33/00

(54) **CAPTEUR DE DÉTECTION À TRANSISTOR À HAUTE MOBILITÉ ÉLECTRONIQUE SÉLECTIF D'UN COMPOSANT GAZEUX OU LIQUIDE**
DETEKTIONSSENSOR MIT EINEM SELEKTIVEN TRANSISTOR MIT HOHER ELEKTRONENMOBILITÄT ZUR ERKENNUNG EINES GASFÖRMIGEN ODER FLÜSSIGEN BESTANDTEILS
DETECTION SENSOR COMPRISING A SELECTIVE HIGH-ELECTRON-MOBILITY TRANSISTOR FOR DETECTING A GASEOUS OR LIQUID COMPONENT

(43) Date de publication de la demande: 21.08.2019
(73) Titulaire: Peugeot Citroën Automobiles SA, 78140 Vélizy-Villacoublay (FR); Georgia Tech Lorraine, 57070 Metz (FR); Centre National de la Recherche Scientifique, 75016 Paris (FR)
(72) Inventeur: AUBRY, Vincent, 91400 Orsay (FR); OUGAZZADEN, Abdallah, 51156 Marly (FR); SALVESTINI, Jean-Paul, 57070 Metz (FR); VOSS, Paul, 57000 Metz (FR); HALFAYA, Yacine, 57070 Metz (FR); BISHOP, Chris, 57000 Metz (FR)
(74) Mandataire: IPAZ
(86) Numéro de dépôt international: PCT/EP2017/076377
(87) Numéro de publication internationale: WO 2018/069553

(56) Documents cités:
- FR-A1- 2 985 813
- US-A1- 2011 045 600
- US-A1- 2013 288 378
- US-B2- 7 825 435
- YACINE HALFAYA ET AL: "Investigation of the Performance of HEMT-Based NO, NO2 and NH3 Exhaust Gas Sensors for Automotive Antipollution Systems", SENSORS, vol. 16, no. 3, 23 février 2016 (2016-02-23), page 273, XP055365136, CH ISSN: 1424-8220, DOI: 10.3390/s16030273
- BISHOP CHRIS ET AL: "Experimental Study and Device Design of NO, NO2, and NH3 Gas Detection for a Wide Dynamic and Large Temperature Range Using Pt/AlGaN/GaN HEMT", IEEE SENSORS JOURNAL, IEEE SERVICE CENTER, NEW YORK, NY, US, vol. 16, no. 18, 1 septembre 2016 (2016-09-01), pages 6828-6838, XP011620162, ISSN: 1530-437X, DOI: 10.1109/JSEN.2016.2593050 [extrait le 2016-08-15]

## Description

L'invention concerne un capteur de détection ou de mesure d'un composant spécifique présent dans un mélange gazeux ou liquide, le capteur comportant une cellule de captage à transistor à haute mobilité électronique.

Un transistor à haute mobilité électronique, aussi connu sous l'acronyme anglosaxon de HEMT pour « High Electron Mobility Transistor » comprend une source et un drain avec une grille intercalée entre la source et le drain. Le fonctionnement de la cellule de captage est basé sur la conductance entre deux contacts ohmiques que sont la source et le drain, par l'action électrostatique d'une électrode de commande qui est la grille.

Dans ce qui va suivre, il va être développé une application préférentielle du capteur selon la présente invention comme capteur de détection sélective d'un élément polluant présent dans les gaz d'échappement évacués d'un moteur à combustion interne par une ligne d'échappement de véhicule automobile. Cette application n'est pas limitative de la présente invention.

Il est connu que les gaz d'échappement des véhicules contiennent de nombreux composants chimiques dont certains sont nocifs pour la santé des individus et dont certains le sont pour l'environnement. Pour limiter ces nuisances pour l'environnement et la santé, des réglementations ont été mises en place en Europe, aux Etats-Unis et au Japon pour la mesure et/ou la détection de ces composants chimiques.

Concernant les oxydes d'azote faisant partie de ces composants chimiques dangereux, ci-après désignés par leur formule chimique NO_{X}, les limites d'émission seront de plus en plus sévères et elles seront probablement spécifiques aux différents oxydes d'azote dont NO, NO₂, N₂O, etc., alors qu'actuellement les NO_{X} sont réglementés de manière globale.

Les capteurs actuellement utilisés pour les gaz des échappements automobiles sont les capteurs NOₓ et les sondes O₂, aussi connu sous l'appellation de sonde lambda. Ils sont basés sur le principe de fonctionnement des électrolytes solides et la loi de Nernst, et sont à base d'oxyde de zirconium dopé à l'Yttrium.

Ces capteurs NO_{X} ne sont pas sélectifs entre les différents gaz et détectent une concentration globale correspondant aux gaz NO₂, NO, N₂O, NH₃. De plus, leur temps de réponse est élevé et ces capteurs sont relativement chers.

Différentes autres technologies de capteurs de gaz existent tels que des capteurs optiques, des capteurs métal oxyde, des capteurs acoustiques, des capteurs gravimétriques, etc. Mais aujourd'hui, il n'existe pas de capteurs sensibles, rapides, à bas coût, résistants à l'environnement d'un échappement automobile et sélectif aux différentes espèces gazeuses telles que O₂, H₂, NO₂, NO, N₂O, CO, CO₂.

Une nouvelle génération de capteurs sélectifs entre les gaz est donc nécessaire pour être conforme à cette évolution des réglementations. De plus, un capteur permettant de déterminer séparément la concentration d'ammoniac ou NH₃ et le ratio NO/NO₂ permettrait d'améliorer la régulation d'un système de réduction catalytique sélective aussi connu sous l'abréviation RCS réduisant les NOₓ par injection de NH₃ résultant de la décomposition d'un agent réducteur sous forme initiale d'un mélange à base d'urée.

Dans ce contexte, il a été conduit des travaux concernant le développement de capteurs de gaz à base de semi-conducteurs nitrure (III-N). Les semi-conducteurs à base de matériaux III-N sont des matériaux à large bande interdite ce qui les rend intéressants pour des applications de capteur de gaz. Leur stabilité thermique et leur tension de claquage élevée les rendent adaptés à des applications à haute température, ce qui est le cas par exemple pour les lignes d'échappement et/ou les moteurs à combustion interne pour des véhicule automobiles.

Une telle mesure des gaz d'échappement avec des capteurs apporte des avantages essentiels. En effet, l'environnement des gaz d'échappement est très contraint au niveau des très hautes températures rencontrées.

Supprimer les contacts électriques permet d'éviter les contraintes de coûts associés aux connecteurs nécessaires. De plus, la tenue dans l'environnement à haute température de ces connecteurs peut être le facteur limitant en température et non la capacité du capteur lui-même.

Les capteurs peuvent opérer également dans les milieux liquides ou d'accès difficile et peuvent aussi permettre de mesurer des contenus liquides. Cela permet également de réduire l'encombrement des capteurs, ceux-ci ne nécessitant plus de connecteurs.

Cependant les capteurs de mesure et/ou de détection d'un composant à l'intérieur d'un mélange liquide ou gazeux peuvent être améliorés notamment en ce qui concerne la sélectivité du composant à détecter devant concerner uniquement ce composant.

Le document Yacine HALFAYA et AL. "Investigation of the performance of HEMT-based NO, NO2 and NH3 Exhaust Gas Sensors for Automotive Antipollution Systems" décrit des transistors HEMT sensibles aux gaz NO, NO₂ and NH₃. La grille du transistor HEMT est fonctionnalisée à base de platine pour la détection des gaz. Le document BISHOP CHRIS et AL. "Experimental Study and device Design of NO, NO2 and NH3 Gas Détection for a Wide Dynamic and Large Température range Using Pt/AIGaN/GaN HEMT" décrit un capteur de gaz à base de transistor HEMT AIGaN/GaN conçu pour permettre la détection de NO, NO₂ et NH₃ entre 100 ° C et 400 ° C. Une fine couche de platine est utilisée defaçon améliorer la sensibilité. Le document US 2011/045600 A1 décrit un capteur de dioxyde de carbone comprenant un transistor HEMT doté d'une couche de détection sélective sur une partie de la grille, cette couche comprend un polymère contenant des groupes amino. Le document FR2985813 A1 décrit un capteur d'espèces chimiques pour détecter une ou plusieurs espèces chimiques présentes dans un gaz, un liquide ou en phase vapeur. Ce capteur est à base de transistor et comprend des moyens pour alimenter les électrodes. Le document US-A-2013/0288378 divulgue un capteur ou des capteurs servant à la détection et à la mesure d'un ou de plusieurs composants dans un environnement chimique. Ce ou ces capteurs sont basés sur une structure à semiconducteurs comportant une région interfaciale contenant un gaz électronique bidimensionnel. Un catalyseur, réagissant au(x) composant(s), est mis en contact avec la structure à semiconducteurs. Des particules dépouillées provenant du composant ou des composants à l'aide du catalyseur passivent la surface de la structure à semi-conducteurs au niveau de l'interface entre le catalyseur et la structure, provoquant de ce fait un changement de densité de charge dans le gaz à proximité du catalyseur.

Lorsque cette structure de base est incorporée dans un dispositif électronique, tel qu'un transistor à grande mobilité d'électrons ou qu'une diode Schottky, le changement de densité de charge se manifeste par un changement de réponse électrique du dispositif. Par exemple, dans un transistor à haute mobilité électronique, le changement de densité de charge se manifeste sous la forme d'un changement de courant à travers le transistor, et, dans une diode Schottky, le changement de densité de charge se manifeste sous la forme d'un changement de capacité. Par contre, ce document ne divulgue ni ne suggère comment il serait possible de détecter spécifiquement un composant par un tel dispositif.

Par conséquent, le problème à la base de la présente est, pour un capteur de détection et/ou de mesure d'un composant contenu dans un mélange gazeux ou liquide ou liquide, le capteur comprenant une cellule de captage avec un transistor à haute mobilité électronique, de détecter et/ou de mesurer avec précision ce composant contenu de manière sélective par rapport aux autres composants différents présents dans le mélange gazeux ou liquide.

Pour atteindre cet objectif, il est prévu selon l'énoncé de la revendication indépendante 1 un procédé de détection ou de mesure d'au moins un composant spécifique parmi plusieurs composants présents dans un mélange gazeux ou liquide par un capteur comprenant au moins une cellule de captage à transistor comportant une source et un drain avec une grille intercalée entre source et drain, une tension étant appliquée entre source et drain et une intensité de courant dans la cellule de captage étant relevée, où il est procédé à un pilotage de la tension entre source et drain faisant varier l'intensité de courant, le pilotage de la tension se faisant selon un modèle de tension prédéterminé par expérience pour délivrer un profil d'intensité caractéristique dudit au moins un composant spécifique, le procédé étant caractérisé en ce que le transistor est un transistor haute mobilité électronique et le modèle de tension prédéterminé est à échelle de tension à haute fréquence.

L'effet technique est d'obtenir un capteur ou un ensemble de capteurs permettant par différentes tensions entre drain et source d'améliorer fortement la sélectivité à un composant à détecter spécifique. Le procédé selon l'invention est très avantageux comparé aux procédés actuels notamment le cycle en température qui nécessite des durées d'établissement beaucoup plus longues.

En utilisant un procédé selon la présente invention, la sélectivité peut être atteinte en moins d'une seconde simplement en contrôlant des capteurs multiples par des tensions entre drain et source différentes ou simplement en pilotant cette tension sur un seul capteur. Ce contrôle permet d'obtenir des capteurs ou matrice de capteurs à transistor à haute mobilité électronique sélectifs et très rapides dans la détection des gaz ou liquides et mesure de gaz ou liquides notamment pour les applications de mesures et contrôles de gaz d'échappement.

Les avantages de la solution sont une sélectivité entre les différents composants, un temps de réponse amélioré et une diminution du coût.

Avantageusement, le profil d'intensité caractéristique dudit au moins un composant spécifique présente une inversion de sens de l'intensité de courant dans le capteur.

Avantageusement, le même transistor du capteur est soumis consécutivement à différents modèles prédéterminés dédiés à des composants spécifiques différents.

Selon l'invention, le modèle de tension prédéterminé est donc à échelle de tension à haute fréquence.

Avantageusement, les composants détectés ou mesurés sont pris, unitairement ou en combinaison, parmi le monoxyde d'azote, le dioxyde d'azote, l'ammoniac, le monoxyde de carbone, le dioxyde de carbone et l'oxygène, ces composants étant contenus dans des gaz évacués par une ligne d'échappement d'un véhicule automobile.

Avantageusement, le procédé de détection ou de mesure d'au moins un composant spécifique parmi plusieurs composants présents dans un mélange gazeux ou liquide comprend aussi un nettoyage du capteur, où il est appliqué une tension de polarisation entre au moins deux des éléments parmi la source, le drain et la grille ou par une électrode intercalée successivement entre deux des éléments parmi la source, le drain et la grille, la tension de polarisation ou la tension de l'électrode étant prédéterminée pour libérer les ions dudit au moins un composant spécifique retenus sur la grille du capteur.

L'application d'une tension fait croître la température de la zone sensible provoquant la libération des ions gaz captés par la zone sensible. Cette libération de la surface en cassant les liens régénère le capteur à sa situation initiale. Typiquement les capteurs à transistor à haute mobilité électronique ont besoin d'une élévation de température de 300°C pour se régénérer. En appliquant une tension sur une faible zone, par exemple la grille, cette élévation de température locale est suffisante sans accroissement de la température du capteur en entier.

Ce nouveau procédé permet une régénération rapide du capteur et ciblé dans le cas d'une matrice de capteurs autorisant une régénération de certains capteurs pendant que d'autres continuent à fonctionner assurant une mesure continue des gaz.

L'invention concerne également un capteur de détection ou de mesure d'au moins un composant spécifique parmi plusieurs composants présents dans un mélange gazeux ou liquide selon l'énoncé de la revendication indépendante 6.

Avantageusement, le capteur comprend des moyens d'imposition d'une tension de régénération entre au moins deux des éléments parmi la source, le drain et la grille ou une électrode en forme de créneaux successifs s'étendant entre successivement deux des éléments parmi la source, le drain et la grille et des moyens d'imposition à l'électrode d'une tension de régénération, la tension de régénération étant suffisante pour effectuer une régénération du capteur par libération des ions dudit au moins un composant spécifique retenus par le capteur.

Avantageusement, l'invention concerne un ensemble d'au moins deux capteurs de détection ou de mesure, chaque capteur détectant un composant spécifique respectif présent dans un mélange gazeux ou liquide, caractérisé en ce que lesdits au moins deux capteurs sont comme précédemment décrit, chacun desdits au moins deux capteurs présentant un modèle sauvegardé de pilotage de la tension et un profil sauvegardé d'intensité caractéristiques dudit au moins un composant spécifique, une détection sélective d'un composant respectif se faisant par chacun desdits au moins deux capteurs.

Avantageusement, l'invention concerne une ligne d'échappement d'un moteur de combustion interne de véhicule automobile, caractérisée en ce qu'elle comprend un tel capteur ou un tel ensemble d'au moins deux capteurs de détection ou de mesure, le mélange gazeux ou liquide étant formé par des gaz d'échappement traversant la ligne d'échappement et ledit au moins un composant spécifique ou lesdits au moins deux composants spécifiques étant respectivement un ou des polluants contenus dans les gaz d'échappement.

D'autres caractéristiques, buts et avantages de la présente invention apparaîtront à la lecture de la description détaillée qui va suivre et au regard des dessins annexés donnés à titre d'exemples non limitatifs et sur lesquels :
- la figure 1 est une représentation schématique d'un mode de réalisation d'un capteur selon la présente invention, le capteur présentant une cellule de captage comprenant un transistor à haute mobilité électronique,
- la figure 2 montre des courbes d'intensité de courant en fonction du voltage pour différents composants tels que le monoxyde d'azote NO, le dioxyde d'azote NO2 et l'ammoniac NH3,
- les figures 3 et 3a montrent respectivement pour des tensions de 2Volts et de 7 Volts en fonction du temps le courant mesuré pour la détection de dioxyde d'azote,
- les figures 4a, 4b et 4c montrent des courbes de courant en milliampères en fonction du temps en minutes de réponse au monoxyde d'azote pour différents voltages respectivement de 2, 5 et 7 Volts,
- les figures 5 et 5a sont relatives à une régénération préférentielle dans le cadre de la présente invention d'une surface de travail du capteur utilisant une électrode de régénération,
- la figure 6 illustre une régénération préférentielle dans le cadre de la présente invention de la surface de travail du capteur alternative à celle des figures 5 et 5a, la régénération se faisant par tension de régénération,
- la figure 7 montre deux courbes de tension obtenues en fonction de l'épaisseur de la couche de platine de la grille d'un capteur selon la présente invention pour un composant sous forme d'hydrogène et d'ammoniac, le voltage étant représentatif de la sensibilité du capteur par rapport à ces deux éléments,
- la figure 8 montre plusieurs morphologies de couche de platine formant grille dans un capteur selon la présente invention.

Il est à garder à l'esprit que les figures sont données à titre d'exemples et ne sont pas limitatives de l'invention. Elles constituent des représentations schématiques de principe destinées à faciliter la compréhension de l'invention et ne sont pas nécessairement à l'échelle des applications pratiques. En particulier, les dimensions des différents éléments illustrés ne sont pas représentatives de la réalité.

Dans ce qui va suivre, il est fait référence à toutes les figures prises en combinaison. Quand il est fait référence à une ou des figures spécifiques, ces figures sont à prendre en combinaison avec les autres figures pour la reconnaissance des références numériques désignées.

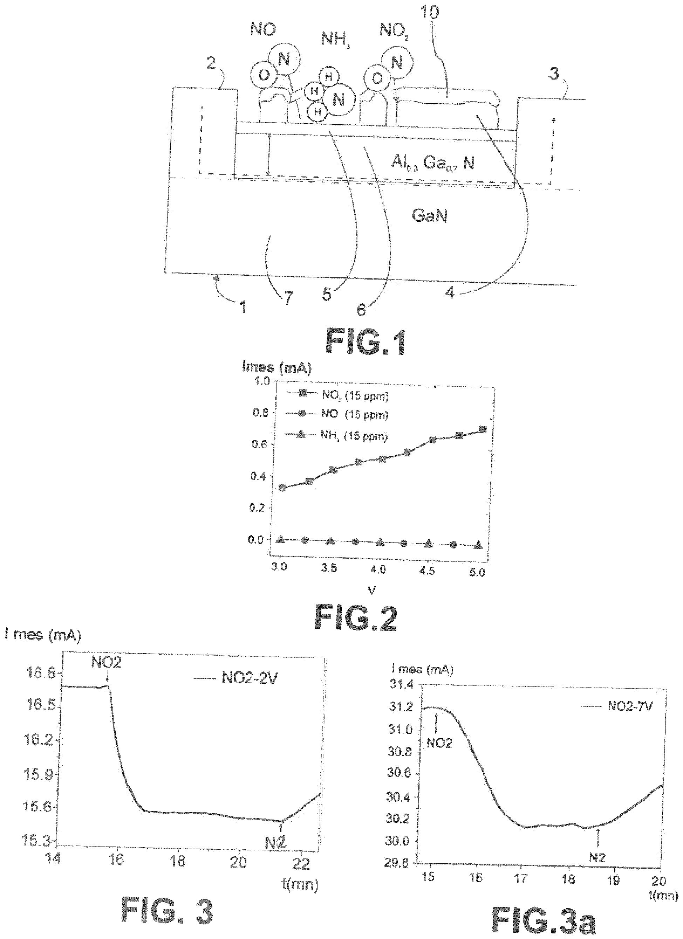

En se référant à toutes les figures et notamment la figure 1, la présente invention concerne un capteur 1 de détection ou de mesure d'au moins un composant spécifique présent dans un mélange gazeux ou liquide. Le capteur 1 comprend au moins une cellule de captage à transistor à haute mobilité électronique comportant une source 2 et un drain 3 avec une grille 4 intercalée entre la source 2 et le drain 3.

Un transistor à haute mobilité électronique porte le drain 3 et la source 2 à deux extrémités latérales opposées. Dans un mode de réalisation, entre la source 2 et le drain 3 s'étendent une couche semi-conductrice 5 III-N nanostructurée et une couche de Al_{0,3}Ga_{0,7}N 6 ou couche active d'interaction électrostatique, la couche semi-conductrice 5 étant superposée à la couche de Al_{0,3}Ga_{0,7}N 6. La couche de Al_{0,3}Ga_{0,7}N 6 est superposée à une couche de GaN 7.

La couche semi-conductrice 5 III-N nanostructurée supporte une 4 ou des couches formant porte d'entrée pour les ions du ou des composants à détecter ou à mesurer, par exemple des ions oxygène négatifs O²⁻ dissociés pour des oxydes d'azote NOx ou de l'oxygène O2 en créant une différence de potentiel. Cette couche 4 ou ces couches formant porte d'entrée, avantageusement revêtues d'une couche d'oxydes 10, peuvent être en platine ou en tungstène.

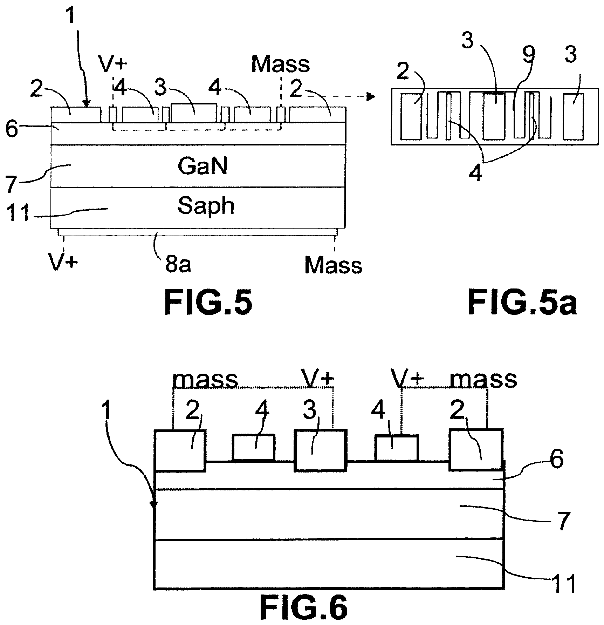

Sous la couche de Al_{0,3}Ga_{0,7}N 6 s'étend un passage reliant la source 2 et le drain 3, le passage étant lui-même superposé à une couche de GaN 7, servant de substrat isolant. A la figure 5, il est prévu une couche de saphir 11 en dessous de la couche de GaN 7.

Le capteur 1 comprend un microprocesseur avec des moyens d'application d'une tension entre source 2 et drain 3 et des moyens de suivi de l'intensité de courant Imes dans la cellule de captage. Selon la présente invention, le microprocesseur est équipé de moyens de pilotage de la tension selon un modèle de tension sauvegardé par des moyens de mémorisation, des moyens de relevé de l'intensité de courant Imes lors de l'application du modèle de tension et des moyens de reconnaissance d'un profil d'intensité caractéristique dudit au moins un composant spécifique sauvegardé par les moyens de mémorisation.

La présente invention concerne aussi un procédé de détection ou de mesure d'au moins un composant spécifique parmi plusieurs composants présents dans un mélange gazeux ou liquide par un capteur 1 comme précédemment décrit. Une tension est appliquée entre source 2 et drain 3 et une intensité de courant Imes dans la cellule de captage est relevée.

Selon l'invention, il est procédé à un pilotage de la tension entre source 2 et drain 3 faisant varier l'intensité de courant Imes, le pilotage de la tension se faisant selon un modèle de tension prédéterminé par expérience pour délivrer un profil d'intensité caractéristique dudit au moins un composant spécifique.

Le même transistor du capteur 1 peut être soumis consécutivement à différents modèles prédéterminés dédiés à des composants spécifiques différents, ceci donc en changeant les tensions appliquées entre la source 2 et le drain 3, tensions qui ont fait l'objet de l'élaboration prédéterminée d'un modèle de tension. Ce modèle de tension prédéterminé est selon l'invention à échelle de tension à haute fréquence.

Une application préférentielle mais non limitative de la présente invention est pour la détection ou la mesure des composants suivants, pris unitairement ou en combinaison : le monoxyde d'azote NO, le dioxyde d'azote NO2, l'ammoniac NH3, le monoxyde de carbone CO, le dioxyde de carbone CO2 et l'oxygène O2. Ces composants ne sont pas limitatifs et sont les principaux contenus dans des gaz évacués par une ligne d'échappement d'un véhicule automobile.

La figure 2 montre des courbes d'intensité de courant Imes en milliampères en fonction du voltage en volts pour différents composants tels que le monoxyde d'azote NO, le dioxyde d'azote NO2 et l'ammoniac NH3, ceci pour 15ppm et 300°C de température.

Le profil d'intensité caractéristique dudit au moins un composant spécifique peut présenter une inversion de sens de l'intensité de courant Imes dans le capteur 1. Ceci est par exemple le cas pour la détection d'un composant gazeux dans la ligne d'échappement d'un véhicule automobile qui est le monoxyde d'azote ou NO. Le courant de signal pour NO change de sens dans la structure du capteur 1 à transistor à haute mobilité électronique mais ce changement de signe s'effectue en présence uniquement de NO et non de dioxyde d'azote NO2 ou d'ammoniac NH3.

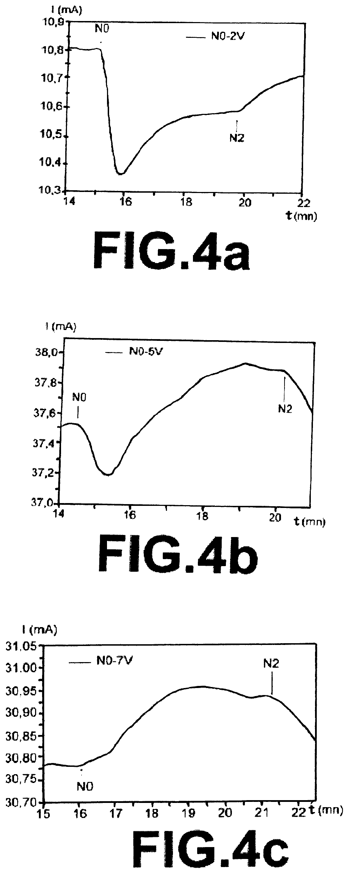

Les figures 3 et 3a montrent respectivement pour des tensions de 2Volts et de 7 Volts le courant mesuré en milliampère mA pour la détection de dioxyde d'azote NO2 en fonction du temps t en minutes. Ces figures 3 et 3a sont à comparer aux figures 4a, 4b et 4c relatives au courant mesuré en milliampères mA pour la détection du monoxyde d'azote NO lorsque le capteur est soumis au monoxyde d'azote, ceci en fonction du temps t en minutes ceci pour différents voltages respectivement de 2, 5 et 7 Volts, la plus grande variation du courant, sous la forme d'une chute brutale se faisant pour 2 Volts. Il peut être vu que l'évolution pour le monoxyde d'azote NO, contrairement au cas du capteur en présence de NO2 aux figures 3 et 3a dépend fortement de la tension de polarisation. Une inversion de l'évolution en passant d'une chute à une montée est observée lorsque la polarisation passe de 2 à 7 Volts.

Le changement de signe du signal NO est donc obtenu en appliquant différentes tensions entre drain 3, source 2 et aussi grille 4. Ceci permet de changer la réaction de la surface chimique. Il s'est avéré qu'un modèle de tension pouvait être élaboré pour de nombreux composants spécifiquement à un composant pris unitairement et que l'élaboration d'un tel modèle pouvait conduire à une détection et une mesure précises du composant par relevé des intensités de courant dans le capteur 1.

Il est possible d'utiliser les changements de signes du signal NO pour améliorer la sélectivité des capteurs 1 aux différents gaz en présence, par exemple NO2, NH3, CO, CO2, O2, etc., dans le cas de composants dans des gaz d'échappement de véhicule automobile.

L'invention concerne aussi un ensemble d'au moins deux capteurs 1 de détection ou de mesure. Chaque capteur 1 détecte un composant spécifique respectif présent dans un mélange gazeux ou liquide. Les deux capteurs 1 peuvent être comme précédemment décrit, chacun desdits au moins deux capteurs 1 présentant un modèle sauvegardé de pilotage de la tension et un profil sauvegardé d'intensité caractéristiques dudit au moins un composant spécifique, une détection sélective d'un composant respectif se faisant par chacun desdits au moins deux capteurs 1.

Un tel capteur 1 présente le désavantage de s'encrasser par dépôt de particules de composants sur sa surface de contact et notamment sur sa grille 4 ou sur la couche d'oxydes 10 recouvrant la grille 4. Dans le cadre du procédé précédemment mentionné pour la détection ou la mesure d'au moins un composant spécifique parmi plusieurs composants présents dans un mélange gazeux ou liquide, il peut être mis en oeuvre un procédé de nettoyage du capteur 1 à transistor à haute mobilité électronique.

Selon un premier mode de réalisation du procédé de régénération selon l'invention, il est appliqué une tension de polarisation par une électrode 9 intercalée successivement entre deux des éléments parmi la source 2, le drain 3 et la grille 4, la tension de l'électrode 9 étant prédéterminée pour libérer les ions dudit au moins un composant spécifique retenus sur la grille 4 du capteur 1. Ceci est montré aux figures 5 et 5a pour une électrode 9.

Selon un deuxième mode de réalisation du procédé de régénération selon l'invention, il est appliqué une tension de polarisation entre au moins deux des éléments parmi la source 2, le drain 3 et la grille 4, la tension de polarisation étant prédéterminée pour libérer les ions dudit au moins un composant spécifique retenus sur la grille 4 du capteur 1. Ceci est montré à la figure 6.

Aux figures 5, 5a et 6, V+ et Mass indiquent les pôles du circuit de régénération. Il peut exister un circuit de régénération 8a auxiliaire à la base du capteur 1.

En se référant notamment à la figure 1, la grille 4 comprend un corps de platine ou de tungstène avantageusement revêtu par une couche d'oxydes 10. Un capteur 1 à transistor à haute mobilité électronique peut avoir de nombreux mécanismes de détection différents qui sont fonction de l'épaisseur de la grille 4, de son matériau et de sa morphologie.

Par exemple, les courbes de la figure 2 montrant une sélectivité entre NO2 et NO/NH3 ont été obtenues en utilisant une fine couche de platine sur des contacts BGaN/GaN de capteur à diode Schottky.

L'épaisseur de la couche de platine formant grille 4 peut être de 100ηm afin de supprimer la présence de pores dans la couche platine, tandis que des grains se forment pendant le processus de croissance de la surface BGaN. L'épaisseur de la couche de la grille 4 a un fort impact sur la sensibilité, soit 0% pour 100 ηm de couche de platine pour le NO, NO2, NH3, contre 20% pour 15 ηm de couche de platine.

Il a aussi été remarqué que le signal de mesure dû a la présence NH3 ou signal NH3 peut s'inverser en changeant de signe en mettant en oeuvre des modifications de la morphologie et de l'épaisseur de grille 4.

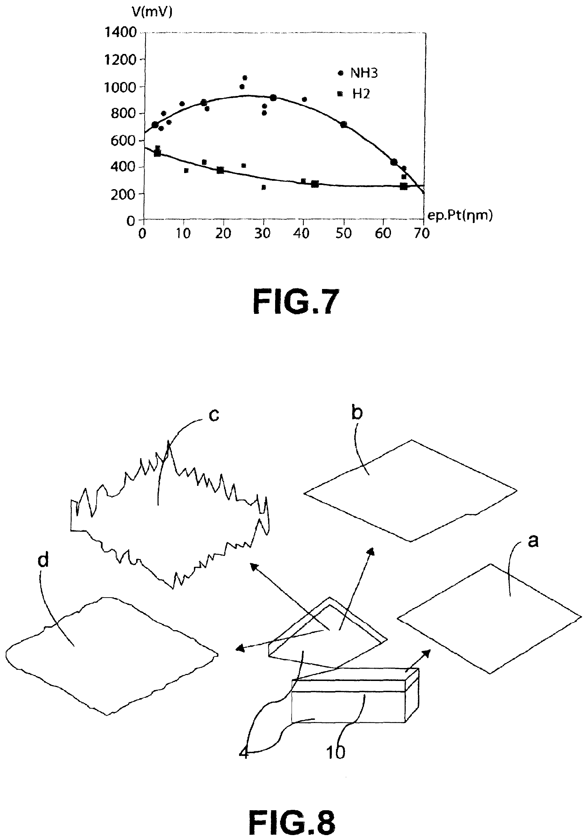

La figure 7 montre deux courbes d'une tension en millivolts mV par rapport à une épaisseur de grille 4 en couche de platine en nanomètre ηm pour de l'hydrogène H2 et de l'ammoniac NH3 à 150°C avec comme gaz porteur 20% d'oxygène dans de l'argon. La sensibilité du capteur étant fonction de la tension, il peut être vu à cette figure 7 que la sensibilité est dépendante de l'épaisseur de la grille 4.

La figure 8 montre une morphologie de la grille 4 différent selon le procédé de fabrication de la grille 4. La grille 4 sous forme d'une couche de platine peut être obtenue par évaporation référencée d à la figure 8, par pulvérisation sous une première pression référencée b, par pulvérisation sous une deuxième pression référencée c ou revêtue d'une couche d'oxydes référencée a avec un dépôt par masquage du platine en nanocolonnes ou autres structures géométriques.

Ces modifications peuvent s'appliquer à toutes les structures de capteurs 1 à transistor à haute mobilité électronique ou à la grille 4 sous forme d'une diode Schottky afin d'améliorer la sensibilité et la sélectivité.

## Revendications

1. Procédé de détection ou de mesure d'au moins un composant spécifique parmi plusieurs composants présents dans un mélange gazeux ou liquide par un capteur (1) comprenant au moins une cellule de captage à transistor comportant une source (2) et un drain (3) avec une grille (4) intercalée 5 entre source (2) et drain (3), une tension étant appliquée entre source (2) et drain (3) et une intensité de courant (Imes) dans la cellule de captage étant relevée, où il est procédé à un pilotage par différentes tensions de la tension entre source (2) et drain (3) faisant varier l'intensité de courant (Imes), le pilotage de la tension se faisant selon un modèle de tension prédéterminé par expérience pour délivrer un profil d'intensité caractéristique dudit au moins un composant spécifique,
le procédé étant **caractérisé en ce que** le transistor est un transistor haute mobilité électronique et le modèle de tension prédéterminé est à échelle de tension à haute fréquence.

2. Procédé selon la revendication précédente, dans lequel le profil d'intensité caractéristique dudit au moins un composant spécifique présente une inversion de sens de l'intensité de courant (Imes) dans le capteur (1).

3. Procédé selon l'une quelconque des revendications précédentes, dans lequel le même transistor du capteur (1) est soumis consécutivement à différents modèles prédéterminés dédiés à des composants spécifiques différents.

4. Procédé selon l'une quelconque des revendications précédentes, dans lequel les composants détectés ou mesurés sont pris, unitairement ou en combinaison, parmi le monoxyde d'azote, le dioxyde d'azote, l'ammoniac, le monoxyde de carbone, le dioxyde de carbone et l'oxygène, ces composants étant contenus dans des gaz évacués par une ligne d'échappement d'un véhicule automobile.

5. Procédé selon l'une des revendications précédentes, **caractérisé en ce qu'**il comprend un nettoyage du capteur (1), où le nettoyage comprend l'application d'une tension de polarisation entre au moins deux des éléments parmi la source (2), le drain (3) et la grille (4) ou par une électrode (9) intercalée successivement entre deux des éléments parmi la source (2), le drain (3) et la grille (4), la tension de polarisation ou la tension de l'électrode (9) étant prédéterminée pour libérer les ions dudit au moins un composant spécifique retenus sur la grille (4) du capteur (1).

6. Capteur (1) de détection ou de mesure d'au moins un composant spécifique parmi plusieurs composants présents dans un mélange gazeux ou liquide, le capteur (1) comprenant au moins une cellule de captage à transistor comportant une source (2) et un drain (3) avec une grille (4) intercalée entre source (2) et drain (3), le capteur (1) comprenant un microprocesseur avec des moyens d'application d'une tension entre source (2) et drain (3) et des moyens de suivi de l'intensité de courant (Imes) dans la cellule de captage, où le microprocesseur est équipé :
- de moyens de pilotage par différentes tensions de la tension selon un modèle de tension sauvegardé par des moyens de mémorisation,
- des moyens de relevé de l'intensité de courant (Imes) lors de l'application du modèle de tension et
- des moyens de reconnaissance d'un profil d'intensité caractéristique dudit au moins un composant spécifique sauvegardé par les moyens de mémorisation,
le capteur (1) étant **caractérisé en ce que** le transistor est un transitor à haute mobilité électronique et le modèle de tension sauvegardé est un modèle à échelle de tension à haute fréquence.

7. Capteur (1) selon la revendication 6, lequel comprend des moyens d'imposition d'une tension de régénération entre au moins deux des éléments parmi la source (2), le drain (3) et la grille (4) ou une électrode (9) en forme de créneaux successifs s'étendant entre successivement deux des éléments parmi la source (2), le drain (3) et la grille (4) et des moyens d'imposition à l'électrode (9) d'une tension de régénération, la tension de régénération étant suffisante pour effectuer une régénération du capteur (1) par libération des ions dudit au moins un composant spécifique retenus par le capteur (1).

8. Ensemble d'au moins deux capteurs (1) de détection ou de mesure, chaque capteur (1) détectant un composant spécifique respectif présent dans un mélange gazeux ou liquide, **caractérisé en ce que** lesdits au moins deux capteurs (1) sont selon l'une quelconque des revendications 6 ou 7, chacun desdits au moins deux capteurs (1) présentant un modèle sauvegardé de pilotage de la tension et un profil sauvegardé d'intensité caractéristiques dudit au moins un composant spécifique, une détection sélective d'un composant respectif se faisant par chacun desdits au moins deux capteurs (1).

9. Ligne d'échappement d'un moteur de combustion interne de véhicule automobile, **caractérisée en ce qu'**elle comprend au moins un capteur (1) selon l'une quelconque des revendications 6 ou 7 ou au moins un ensemble d'au moins deux capteurs (1) de détection ou de mesure selon la revendication 8, le mélange gazeux ou liquide étant formé par des gaz d'échappement traversant la ligne d'échappement et ledit au moins un composant spécifique ou lesdits au moins deux composants spécifiques étant respectivement un ou des polluants contenus dans les gaz d'échappement.

## Patentansprüche

1. Verfahren zum Nachweis oder zur Messung mindestens einer spezifischen Komponente unter mehreren Komponenten, die in einer gasförmigen oder flüssigen Mischung vorhanden sind, durch einen Sensor (1), der mindestens eine transistorbasierte Sensorzelle umfasst mit einer Quelle (2) und einem Abfluss (3) mit einem zwischen Quelle (2) und Abfluss (3) eingefügten Gate (4), wobei zwischen Quelle (2) und Abfluss (3) eine Spannung angelegt wird und eine Stromstärke (lmes) in der Sensorzelle erfasst wird, wobei durch verschiedene Spannungen die Spannung zwischen Quelle (2) und Abfluss (3) gesteuert wird, was zu einer Variation der Stromstärke (lmes) führt und wobei die Steuerung der Spannung gemäß einem durch Erfahrung vorgegebenen Spannungsmuster erfolgt, um ein für die mindestens eine spezifische Komponente charakteristisches Stromstärkeprofil zu liefern,
wobei das Verfahren **dadurch gekennzeichnet ist, dass** der Transistor ein Transistor mit hoher Elektronenbeweglichkeit ist und das vorgegebene Spannungsmuster mit einer hochfrequenten Spannungsskala ist.

2. Verfahren nach dem vorhergehenden Anspruch, bei dem das charakteristische Stromstärkeprofil der mindestens einen spezifischen Komponente eine Richtungsumkehrung der Stromstärke (lmes) im Sensor (1) aufweist.

3. Verfahren nach einem der Ansprüche, bei dem derselbe Transistor des Sensors (1) nacheinander verschiedenen vorbestimmten Mustern, die verschiedenen spezifischen Komponenten zugeordnet sind, unterzogen wird.

4. Verfahren nach einem der vorhergehenden Ansprüche, bei dem die erfassten oder gemessenen Komponenten einzeln oder in Kombination aus Stickstoffmonoxid, Stickstoffdioxid, Ammoniak, Kohlenmonoxid, Kohlendioxid und Sauerstoff ausgewählt werden, wobei diese Komponenten in Gasen enthalten sind, die aus einer Abgasleitung eines Kraftfahrzeugs ausgestoßen werden.

5. Verfahren nach einem der vorhergehenden Ansprüche, **dadurch gekennzeichnet, dass** es eine Reinigung des Sensors (1) umfasst, wobei die Reinigung das Anlegen einer Polarisationsspannung zwischen mindestens zwei der Elemente aus Quelle (2), Abfluss (3) und Gate (4) umfasst oder durch eine Elektrode (9), die nacheinander zwischen zwei der Elemente aus Quelle (2), Abfluss (3) und Gate (4) eingefügt wird, erfolgt, wobei die Polarisationsspannung oder die Spannung der Elektrode (9) vorbestimmt ist, um die Ionen der mindestens einen spezifischen Komponente, die am Gate (4) des Sensors (1) festgehalten werden, freizusetzen.

6. Sensor (1) zum Nachweis oder Messen mindestens einer bestimmten Komponente aus einer Vielzahl von Komponenten, die in einem gasförmigen oder flüssigen Gemisch vorhanden sind, wobei der Sensor (1) mindestens eine transistorbasierte Sensorzelle umfasst mit einer Quelle (2) und einem Abfluss (3) und mit einem zwischen Quelle (2) und Abfluss (3) eingefügten Gate (4), wobei der Sensor (1) einen Mikroprozessor mit Mitteln zum Anlegen einer Spannung zwischen Quelle (2) und Abfluss (3) und Mittel zum Verfolgen der Stromstärke (lmes) in der Sensorzelle umfasst, wobei der Mikroprozessor ausgestattet ist mit:
- Mitteln zur Steuerung durch verschiedene Spannungen der Spannung nach einem durch Speichermittel gesicherten Spannungsmuster,
- Mittel zum Erfassen der Stromstärke (lmes) beim Anlegen des Spannungsmusters und
- Mittel zur Erkennung eines charakteristischen Stomstärkeprofils der mindestens einen spezifischen Komponente, das von den Speichermitteln gespeichert wird, wobei der Sensor (1) **dadurch gekennzeichnet ist, dass** der Transistor ein Transitor mit hoher Elektronenbeweglichkeit ist und das gespeicherte Spannungsmuster eine hochfrequente Spannungsskala ist.

7. Sensor (1) nach Anspruch 6, der Mittel zum Anlegen einer Regenerationsspannung zwischen mindestens zwei der Elemente aus Quelle (2), Abfluss (3) und Gate (4) oder eine Elektrode (9) in Form von aufeinanderfolgenden U-Marken umfasst, die sich nacheinander zwischen zwei der Elemente aus Quelle (2), Abfluss (3) und Gate (4) erstrecken, und Mittel zum Anlegen einer Regenerationsspannung an der Elektrode (9), wobei die Regenerationsspannung ausreicht, um eine Regeneration des Sensors (1) durch Freisetzung der Ionen der mindestens einen spezifischen Komponente, die von dem Sensor (1) festgehalten werden, zu bewirken.

8. Anordnung von mindestens zwei Sensoren (1) zum Nachweis oder Messen, wobei jeder Sensor (1) die eine jeweilige spezifische Komponente erfasst, die in einer gasförmigen oder flüssigen Mischung vorhanden ist, **dadurch gekennzeichnet, dass** die mindestens zwei Sensoren (1) gemäß einem der Ansprüche 6 oder 7 sind, wobei jeder der mindestens zwei Sensoren (1) ein gespeichertes Spannungssteuerungsprofil und ein gespeichertes Stromstärkeprofil aufweist, die für die mindestens eine spezifische Komponente charakteristisch sind, wobei eine selektive Erfassung einer jeweiligen Komponente durch jeden der mindestens zwei Sensoren (1) erfolgt.

9. Abgasleitung eines Verbrennungsmotors eines Kraftfahrzeugs, **dadurch gekennzeichnet, dass** sie mindestens einen Sensor (1) nach einem der Ansprüche 6 oder 7 umfasst oder mindestens eine Anordnung von mindestens zwei Sensoren (1) zum Nachweis oder Messen nach Anspruch 8, wobei das Gas- oder Flüssigkeitsgemisch durch Abgase gebildet wird, die durch die Abgasleitung strömen, und wobei die mindestens eine spezifische Komponente oder die mindestens zwei spezifischen Komponenten jeweils ein Schadstoff oder Schadstoffe sind, die in den Abgasen enthalten sind.

## Claims

1. A method of detecting or measuring at least one specific component among several components present in a gaseous or liquid mixture by means of a sensor (1) comprising at least one collection cell having a transistor comprising a source (2) and a drain (3) with a gate (4) inserted between source (2) and drain (3), a voltage between source (2) and drain (3) being applied a current intensity (Imeas) in the collection cell being recorded; wherein the voltage between source (2) and drain (3) is driven for different voltages by varying the current intensity (Imeas), the voltage being driven according to a voltage model predetermined by experiment in order to deliver a profile with an intensity which is characteristic of said at least one specific component,
the method being **characterized in that** the transistor is a high-electron-mobility transistor and the predetermined voltage model is on a high-frequency voltage scale.

2. The method according to the preceding claim, in which the profile with an intensity which is characteristic of said at least one specific component has an inversion of sign of the current intensity (Imeas) in the sensor (1).

3. The method according to any one of preceding claims, in which a same transistor of the sensor (1) is consecutively subject to different predetermined models dedicated to different specific components.

4. The method according to any one of preceding claims, in which the components detected or measured are taken, individually or in combination, from among nitrogen monoxide, nitrogen dioxide, ammonia, carbon monoxide, carbon dioxide and oxygen, these components being contained in the gases evacuated by means of an exhaust line of a motor vehicle.

5. The method according to any one of preceding claims, characterized in thatit comprises a cleaning of the sensor (1), wherein that the cleaning comprises applying a polarization voltage between at least two of the elements between the source (2), the drain (3) and the gate (4) or by an electrode (9) inserted successively between two of the elements from among the source (2), the drain (3) and the gate (4), the polarization voltage or the voltage of the electrode (9) being predetermined in order to release the ions of said at least one specific component retained on the gate (4) of the sensor (1).

6. A sensor (1) for detecting or measuring at least one specific component from among several components present in a gaseous or liquid mixture, the sensor (1) comprising at least one collection cell having a transistor comprising a source (2) and a drain (3) with a gate (4) inserted between the source (2) and the drain (3), the sensor (1) comprising a microprocessor with means for applying a voltage between the source (2) and the drain (3) and means for monitoring the current intensity (Imeas) in the collection cell, wherein the microprocessor is equipped with
- voltage drive means for driving the voltage by different voltages according to a voltage model saved by storage means,
- means for recording the current intensity (Imeas) during application of the voltage model and
- means for recognizing an intensity profile characteristic of said at least one specific component saved by the storage means, the sensor (1) being **characterized in that** the transistor is a high-electron-mobility transistor and the saved voltage model is a high-frequency voltage scale model.

7. The sensor (1) according to claim 6, which comprises means for imposing a regeneration voltage between at least two of the elements from among the source (2), the drain (3) and the gate (4) or an electrode (9) in the shape of successive slots extending successively between two of the elements from among the source (2), the drain (3) and the gate (4) and means for imposing a regeneration voltage to the electrode (9), the regeneration voltage being sufficient to regenerate the sensor (1) by releasing ions of said at least one specific component retained by the sensor (1)..

8. Assembly of at least two detection or measuring sensors (1), each sensor (1) detecting a respective specific component present in a gaseous or liquid mixture, **characterized in that** said at least two sensors (1) are according to any one of claims 6 or 7, each of said at least two sensors (1) having a stored voltage drive model and a stored intensity profile which is characteristic of said at least one specific component, a selective detection of a respective component being made by each of said at least two sensors (1).

9. Exhaust line of an internal combustion engine of a motor vehicle, **characterized in that** it comprises at least one sensor (1) according to any one of claims 6 or 7 or at least an assembly of at least two detection or measurement sensors (1) according to claim 8, the gaseous or liquid mixture being formed by exhaust gases passing through the exhaust line and said at least one specific component or at least two specific components being respectively one or more pollutants contained in the exhaust gas.
